# EUROPÄISCHE PATENTSCHRIFT

(11) **EP 0 865 498 B1**
(45) Veröffentlichungstag und Bekanntmachung des Hinweises auf die Patenterteilung: **23.10.2002**
(21) Anmeldenummer: 96939916.1
(22) Anmeldetag: 25.11.1996
(51) Int. Cl.: C12P 17/14, C07D 273/00

(54) **VERFAHREN ZUR HERSTELLUNG VON SUBSTITUIERTEN ARYLMILCHSÄURE-HALTIGEN CYCLODEPSIPEPTIDEN MIT 24 RINGATOMEN**
PROCESS FOR THE PREPARATION OF SUBSTITUTED ARYL LACTIC ACID CONTAINING CYCLODEPSIPEPTIDES WITH 24 RING ATOMS
PROCEDE DE PRODUCTION DE CYCLODEPSIPEPTIDES SUBSTITUES CONTENANT DE L'ACIDE ARYLLACTIQUE ET COMPORTANT 24 ATOMES DE CYCLE

(30) Priorität: 07.12.1995 DE 19545639
(43) Veröffentlichungstag der Anmeldung: 23.09.1998
(73) Patentinhaber: BAYER AG, 51368 Leverkusen (DE)
(72) Erfinder: JESCHKE, Peter, D-51373 Leverkusen (DE); BONSE, Gerhard, D-51061 Köln (DE); THIELKING, Gerhard, D-51379 Leverkusen (DE); ETZEL, Winfried, D-42799 Leichlingen (DE); HARDER, Achim, D-51109 Köln (DE); MENCKE, Norbert, D-51381 Leverkusen (DE); KLEINKAUF, Horst, D-12205 Berlin (DE); ZOCHER, Rainer, D-12169 Berlin (DE); IINUMA, Katsuharu, Odawara-shi, Kanagawa-ken 250 (JP); MIYAMOTO, Kouichi, Odawara-shi, Kanagawa-ken 250 (JP)
(86) Internationale Anmeldenummer: EP9605190
(87) Internationale Veröffentlichungsnummer: WO97020945

(56) Entgegenhaltungen:
- EP-A- 0 288 087
- EP-A- 0 382 173
- DATABASE WPI Section Ch, Week 9431 Derwent Publications Ltd., London, GB; Class B03, AN 94-252800 XP002026183 & JP 06 184 126 A (MEIJI SEIKA KAISHA) , 5.Juli 1994 in der Anmeldung erwähnt
- CHEMICAL ABSTRACTS, vol. 117, no. 7, 17.August 1992 Columbus, Ohio, US; abstract no. 62356, SASAKI, TORU ET AL: "A new anthelmintic cyclodepsipeptide, PF1022A" XP002026182 & J. ANTIBIOT. (1992), 45(5), 692-7 CODEN: JANTAJ;ISSN: 0021-8820,
- BIOSCI., BIOTECHNOL., BIOCHEM. (1993), 57(1), 98-101 CODEN: BBBIEJ, XP002026181 KAWAZU, KAZUYOSHI ET AL: "Isolation and characterization of two novel nematicidal depsipeptides from an imperfect fungus, strain D1084"

## Beschreibung

Die vorliegende Erfindung betrifft ein neues Verfahren zur Herstellung von teilweise bekannten substituierten Arylmilchsäure-haltigen Cyclodepsipeptiden mit 24 Ringatomen.

Die Herstellung verschiedener cyclischer, Arylmilchsäure-haltiger Depsipeptide mit 24 Ringatomen nach mikrobiellen Verfahren, wie beispielsweise PF 1022A, PF 1022 B, PF 1022 C, PF 1022D und PF 1022E, ist bereits beschrieben (vgl. Fermentation von Cyclooktadepsipeptiden: PF 1022A aus Mycelia sterilia (FERM BP-2671; frühere Bezeichnung FERM P-10 504) in EP-OS 382 173; T. Sasaki et al., J. Antibiotics 45, 1992, S. 692-697; aus derselben Kultur wurden isoliert: PF 1022B, PF 1022C und PF 1022D: JP-Pat. 5 170 749; PF 1022E: JP-Pat. 6 184 126).

Die Verbindungen der PF 1022-Reihe besitzen die folgende Formel (I):

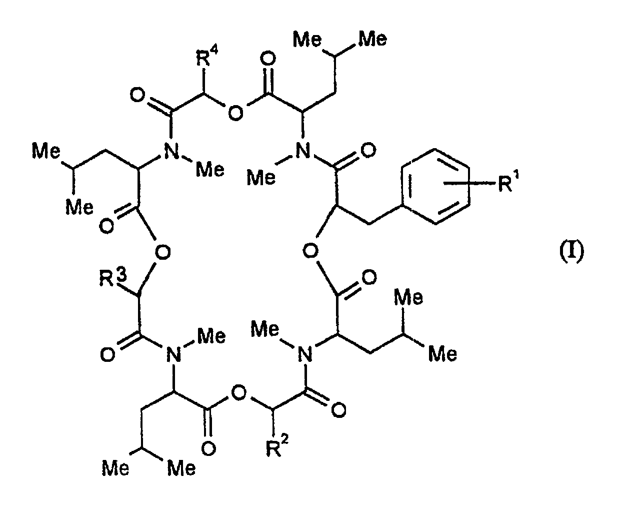

| **Bezeichnung** | **R**^{***1***} | **R**^{***2***} | **R**^{***3***} | **R**^{***4***} |
|---|---|---|---|---|
| PF 1022A | -H | -Methyl | -Benzyl | -Methyl |
| PF 1022B | -H | -Benzyl | -Benzyl | -Benzyl |
| PF 1022C | -H | -Methyl | -Benzyl | -Benzyl |
| PF 1022D | -H | -Methyl | -Methyl | -Methyl |
| PF 1022E | 4-Hydroxy | -Methyl | -Benzyl | -Methyl |

Es ist auch bereits bekannt, daß eine Reihe von cyclischen, Arylmilchsäure-haltigen Depsipeptiden mit 24 Ringatomen mittels chemisch synthetischen Verfahren darstellbar sind (vgl. Totalsynthesen von Cyclooktadepsipeptiden: JP-Pat. 5 229 997; JP-Pat. 5 320 1 48; Makoto Ohyama et al., Biosci. Biotech. Biochem. 58 (6), 1994, S. 1193-1194; Makio Kobayshi et al., Annu. Rep. Sankyo Res. Lab. 46, 1994, S. 67-75; Stephen J. Nelson et al., J. Antibiotics 47, (11), 1994, S. 1322-1327; J. Scherkenbeck et al. Tetrahedron 51 (31), 1995, S. 8459-8470 [PF 1022A]; WO 94/19334; WO 95/19053; EP-OS 634 408; EP-OS 626 375; EP-OS 626 376).

Weiterhin ist bekannt, daß bestimmte Arylmilchsäure-haltige Cyclodepsipeptide mit 24 Ringatomen als Endoparasitizide verwendet werden können (vgl. z.B.: EP-OS 382; EP-OS 503 538; WO 93/19053; EP-OS 0 634 408; WO 94/19334; WO 95/07272; EP-OS 626 375; EP-OS 626 376).

Während chemisch synthetische Verfahren eine breite Variation des Substituenten R¹ im Phenylmilchsäurefragment bestimmter Cyclooktadepsipeptide ermöglichen (vgl.: WO 94/19334; WO 95/19053; EP-OS 634 408; EP-OS 626 375; EP-OS 626 376), entstanden bisher bei den mikrobiellen Verfahren nur bevorzugt die unsubstituierten D-Phenylmilchsäure-haltigen Cyclooktadepsipeptide (R¹ = -H; vgl.: PF 1022A, PF 1022B, PF 1022C und PF 1022D).

Außer der bereits genannten Fermentation des D-(4-Hydroxyphenyl)-milchsäurehaltigen Cyclooktadepsipeptids PF 1022E (R¹ = 4-Hydroxy; vgl.: JP-Pat. 6 184 126) ist somit über die fermentative Darstellung von weiteren, substituierten Arylmilchsäure-haltigen Cyclooktadepsipeptiden bisher nichts bekannt geworden.

Die vorliegende Erfindung betrifft daher ein Verfahren zur Herstellung substituierter Arylmilchsäure-haltiger Cyclodepsipeptide mit 24 Ringatomen mit Hilfe von Pilzstämmen der Art Agonomycetales oder daraus isolierten enzymatischsn Präparationen.

Bei dem erfindungsgemäßen Verfahren werden die substituierten Arylmilchsäure-haltigen Cyclodepsipeptide mit 24 Ringatomen der allgemeinen Formel (I) 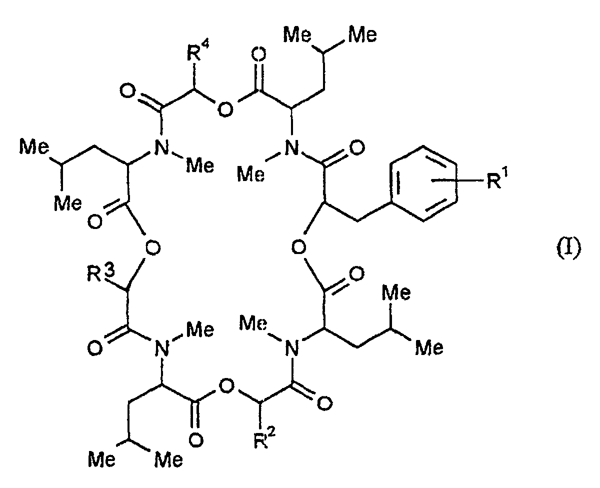 in welcher
- R¹: für geradkettiges oder verzweigtes Alkyl, cyclisches Alkyl, Alkenyl, Alkoxy, Alkenyloxy, Arylalkoxy, Cycloalkoxy, Alkylamino, Dialkylamino, Alkylcarbonyl, Alkylcarbonyloxy, Alkoxycarbonyl, Allylaminocarbonyl, Dialkylaminocarbonyl, Heteroarylcarbonyl, Alkoxysulfonyl, Alkylaminosulfonyl, Dialkylaminosulfonyl, Alkylthio, Alkylsulfinyl, Alkylsulfonyl, Heteroarylsulfonyl, die gegebenenfalls substituiert sein können, Hydroxy, Halogen, Nitro, Amino, Carboxyl, Carbamoyl, Cyano oder gegebenenfalls für substituierte cyclische Aminogruppen, steht, und
- R², R³ und R⁴: unabhängig voneinander für geradkettiges oder verzweigtes Alkyl, Heteroarylmethyl oder für einen Benzylrest stehen, der gegebenenfalls durch Reste aus der Reihe Wasserstoff, geradkettiges oder verzweigtes Alkyl, cyclisches Alkyl, Alkenyl, Alkoxy, Alkenyloxy, Arylalkoxy, Cycloalkoxy, Alkylamino, Dialkylamino, Alkylcarbonyl, Alkylcarbonyloxy, Alkoxycarbonyl, Alkylaminocarbonyl, Dialkylaminocarbonyl, Heteroarylcarbonyl, Alkoxysulfonyl, Alkylaminosulfonyl, Dialkylaminosulfonyl, Alkylthio, Alkylsulfinyl, Alkylsulfonyl, Heteroarylsulfonyl, die gegebenenfalls substituiert sein können, Hydroxy, Halogen, Nitro, Amino, Carboxyl, Carbamoyl, Cyano oder der gegebenenfalls durch eine geeignete cyclische Aminogruppe substituert ist, steht,
mit Ausnahme der Verbindungen der Formel (I), in welcher
- R¹: für 4-Hydroxy steht,
- R³: für unsubstituiertes Benzyl steht
und die übrigen Reste die oben genannte Bedeutung haben, dadurch hergestellt, daß man
a) optisch aktive oder racemische Aminosäuren der allgemeinen Formeln (II), (III), (IV) und (V) 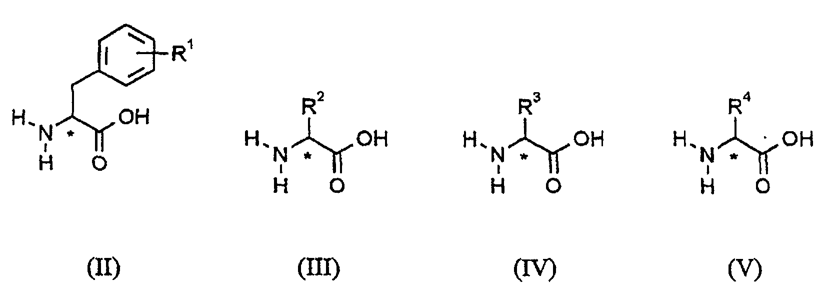 worin
   - R¹, R², R³ und R⁴: die oben angegebene Bedeutung haben, oder
b) optisch aktive oder racemische 2-Hydroxy-carbonsäuren der allgemeinen Formeln (VI), (VII), (VIII) und (IX) 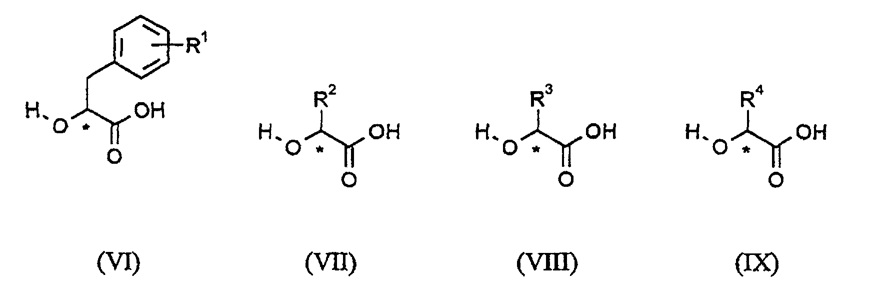 worin
   - R¹, R², R³ und R⁴: die oben angegebene Bedeutung haben,
in Gegenwart von Pilzstämmen der Art Agonomycetales in geeigneten Nährlösungen oder in Gegenwart von aus diesen Pilzstämmen isolierten Synthetasen in einem Puffersystem umsetzt und anschließend die gewünschten, substituierten Arylmilchsäure-haltigen Cyclodepsipeptide mit 24 Ringatomen isoliert.

Die Arylmilchsäure-haltigen cyclischen Depsipeptide mit 24 Ringatomen der allgemeinen Formel (I) sowie deren Säureadditionssalze und Metallsalz-Komplexe eignen sich hervorragend zur Bekämpfung von Endoparasiten, besonders auf dem Gebiet der Medizin und Veterinärmedizin.

Gegebenenfalls substituiertes Alkyl allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl genannt. Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines Restes in den allgemeinen Formeln bedeutet geradkettiges oder verzweigtes Alkenyl mit vorzugsweise 1 bis 6, insbesondere 1 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-pro-penyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-bute-nyl, 1-Ethyl-3-butenyl, 2-Ethyl-2-butenyl, 2-Ethyl-3-butenyl, 1,1,2-Trimethyl-2-prope-nyl, 1-Ethyl-1-methyl-2-propenyl und 1-Ethyl-2-methyl-2-propenyl genannt. Vorzugsweise seien gegebenenfalls substituiertes Ethenyl, 2-Propenyl, 2-Butenyl oder 1-Methyl-2-propenyl genannt.

Gegebenenfalls substituiertes Cycloalkyl allein oder als Bestandteil eines Restes in den allgemeinen Formeln bedeutet mono-, bi- und tricyclisches Cycloalkyl, vorzugsweise mit 3 bis 10, insbesondere mit 3, 5 oder 7 Kohlenstoffatomen. Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]-oktyl und Adamantyl genannt.

Gegebenenfalls substituiertes Alkoxy allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxy, Ethoxy, n-Propoxy, Isopropoxy, n-Butoxy, Isobutoxy, sec-Butoxy und tert-Butoxy genannt.

Gegebenenfalls substituiertes Alkylthio allein oder als Bestandteil eines Restes in den allgemeinen Formeln bedeutet geradkettiges oder verzweigtes Alkylthio mit vorzugsweise 1 bis 6, insbesondere 1 bis 4 Kohlenstoffatomen. Beispielhaft seien gegebenenfalls substituiertes Methylthio, Ethylthio, n-Propylthio, Isopropylthio, n-Butylthio, Isobutylthio, sec-Butylthio und tert-Butylthio genannt.

Gegebenenfalls substituiertes Alkoxycarbonyl allein oder als Bestandteil eines Restes in den allgemeinen Formeln bedeutet geradkettiges oder verzweigtes Alkoxycarbonyl mit vorzugsweise 1 bis 6, insbesondere 1 bis 4 Kohlenstoffatomen. Beispielhaft seien gegebenenfalls substituiertes Methoxycarbonyl, Ethoxycarbonyl, n-Propoxycarbonyl, Isopropoxycarbonyl, n-Butoxycarbonyl, Isobutoxycarbonyl, sec-Butoxycarbonyl und tert-Butoxycarbonyl genannt.

Gegebenenfalls substituiertes Alkylcarbonyl allein oder als Bestandteil eines Restes in den allgemeinen Formeln bedeutet geradkettiges oder verzweigtes Alkylcarbonyl mit vorzugsweise 1 bis 6, insbesondere 1 bis 4 Kohlenstoffatomen. Beispielhaft seien gegebenenfalls substituiertes Methylcarbonyl, Ethylcarbonyl, n-Propylcarbonyl, Isopropylcarbonyl, n-Butylcarbonyl, Isobutylcarbonyl, sec-Butylcarbonyl und tert-Butylcarbonyl genannt.

Gegebenenfalls substituiertes Arylalkyl in den allgemeinen Formeln bedeutet vorzugsweise gegebenenfalls im Arylteil und/oder Alkyl substituiertes Arylalkyl mit vorzugsweise 6 oder 10, insbesondere 8 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls substituiertes Hetaryl allein oder als Bestandteil eines Restes in den allgemeinen Formeln bedeutet 5- bis 7-gliedrige Ringe mit vorzugsweise 1 bis 3, insbesondere 1 oder 2 gleichen oder verschiedenen Heteroaromaten. Als Heteroatome in den Heteroaromaten stehen Sauerstoff, Schwefel oder Stickstoff. Beispielhaft und vorzugsweise seien gegebenenfalls substituiertes Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:
Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio und tert-Butylthio; Halogenalkyl mit vorzugsweise 1 bis 4, insbesondere 1 bis 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethyl-amino, n-Propylamino, Isopropylamino, Methyl-n-butylamino; Alkylcarbonylreste wie Methylcarbonyl; Alkoxycarbonyl mit vorzugsweise 2 bis 4, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogensulfinyl mit 1 bis 4, insbesondere 1 bis 2 Kohlenstoffatomen und 1 bis 5 Halogenatomen wie Trifluormethylsilfinyl; Sulfonyl (-SO₂-OH); Alkylsulfonyl mit vorzugsweise 1 bis 4, insbesondere 1 oder 2 Kohlenstoffatomen, wie Methylsulfonyl und Ethylsulfonyl oder Halogenalkylsulfonyl mit 1 bis 4, insbesondere 1 bis 2 Kohlenstoffatomen und 1 bis 5 Halogenatomen wie Trifluormethylsulfonyl.

Als geeignete cyclische Aminogruppen kommen heteroaromatische oder aliphatische Ringsysteme mit einem oder mehreren Stickstoffatomen als Heteroatom in Frage, bei denen die Heterocyclen gesättigt oder ungesättigt, ein Ringsystem oder mehrere kondensierte Ringsysteme sein können, und gegebenenfalls weitere Heteroatome wie Stickstoff, Sauerstoff und Schwefel u.s.w. enthalten. Außerdem können cyclische Aminogruppen auch ein Spiroring oder ein verbrücktes Ringsystem bedeuten. Die Anzahl der Atome, die cyclische Aminogruppen bilden, ist nicht beschränkt, beispielsweise bestehen sie im Falle eines Einringsystems aus 3 bis 8 Atomen und im Falle eines Dreiringsystems aus 7 bis 11 Atomen.

Beispielhaft für cyclische Aminogruppen seien gesättigte und ungesättigte monocyclische Ringsysteme mit einem Stickstoffatom als Heteroatom wie 1-Azetidinyl, Pyrrolidino, 2-Pyrrolin-1-yl, 1-Pyrrolyl, Piperidino, 1,4-Dihydropyridin-1-yl, 1,2,5,6-Tetrahydropyridin-1-yl, Homopiperidino genannt; beispielhaft für cyclischen Aminogruppen seien gesättigten und ungesättigten monocyclischen Ringsysteme mit zwei oder mehreren Stickstoffatomen als Heteroatome wie 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydropyridazin-1-yl, 1,2-Dihydropyrimidin-1-yl, Perhydropyrimidin-1-yl und 1,4-Diazacycloheptan-1-yl genannt; beispielhaft für cyclischen Aminogruppen seien gesättigten und ungesättigten monocyclischen Ringsysteme mit einem bis drei Stickstoffatomen und einem bis zwei Sauerstoffatomen als Heteroatome wie Oxazolidin-3-yl, Isoxazolin-2-yl, Morpholino oder 2,6-Dimethylmorpholino genannt; beispielhaft für cyclische Aminogruppen seien gesättigte und ungesättigte monocyclische Ringsysteme mit einem bis drei Stickstoffatomen und einem bis zwei Schwefelatomen als Heteroatome genannt, beispielhaft für cyclischen Aminogruppen seien gesättigte und ungesättigte kondensierten cyclische Ringsystemen wie Indol-1-yl, 1,2-Dihydro-benzimidazol-1-yl, Perhydropyrrolo[1,2-a]pyrazin-2-yl genannt; beispielhaft für cyclischen Aminogruppen seien spirocyclische Ringsysteme wie das 2-Azaspiro[4,5]-decan-2-yl genannt; beispielhaft für cyclischen Aminogruppen seien verbrückte heterocyclische Ringsysteme wie das 2-Azabicyclo[2,2,1]heptan-7-yl genannt.

Die erfindungsgemäßen herstellbaren substituierten Arylmilchsäure-haltigen Cyclodepsipeptide mit 24 Ringatomen sind durch die allgemeine Formel (I) allgemein definiert.

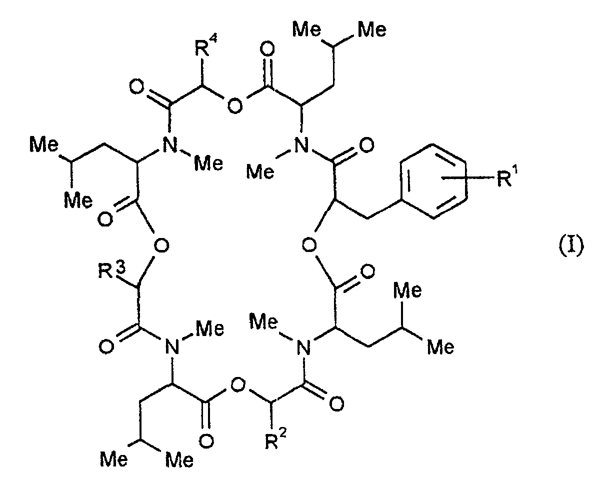

Bevorzugt hergestellt werden Verbindungen der allgemeinen Formel (I),
in welcher
- R¹: für geradkettiges oder verzweigtes Alkyl mit bis zu 4 Kohlenstoffatomen, insbesondere Methyl, Ethyl, Propyl, Isopropyl, Butyl, Isobutyl, sec-Butyl, tert-Butyl, cyclisches Alkyl mit bis zu 6 Kohlenstoffatomen, insbesondere Cyclopropyl, Cyclobutyl, Cyclopentyl, Cyclohexyl, Alkoxy, insbesondere Methoxy, Ethoxy, Propoxy, Isopropoxy, Butoxy, Isobutoxy, sec-Butoxy, tert-Butoxy, Cycloalkoxy, insbesondere Cyclopropyloxy, Alkenyloxy, insbesondere Allyloxy, Dioxoalkylen, insbesondere Dioxomethylen, Alkylamino, insbesondere Methylamino, Ethylamino, Dialkylamino, insbesondere Dimethylamino, Diethylamino, Alkoxycarbonyl, insbesondere Methoxycarbonyl, Ethoxycarbonyl, tert-Butoxycarbonyl, Alkylaminocarbonyl, insbesondere Methylaminocarbonyl, Dialkylaminocarbonyl, insbesondere Dimethylaminocarbonyl, Alkylaminosulfonyl, insbesondere Methylaminosulfonyl, Dialkylaminosulfonyl, insbesondere Dimethylaminosulfonyl, Alkylthio, insbesondere Methylthio oder tert-Butylthio, Alkylsulfinyl, insbesondere Methylsulfinyl oder tert-Butylsulfinyl, Alkylsulfonyl, insbesondere Methylsulfonyl oder tert-Butylsulfonyl, Heteroarylsulfonyl, insbesondere N-Morpholinosulfonyl oder N-Pyrazolylsulfonyl, die gegebenenfalls substituiert sein können, Hydroxy, Halogen, insbesondere Brom, Chlor, Fluor oder Iod, Nitro, Amino, Carboxyl, Carbamoyl, Cyano oder gegebenenfalls für substituierte cyclische Aminogruppen, insbesondere N-Pyrrolidino, N-Piperidino, N-Morpholino, N-(2,6-Dimethyl-morpholino), N-Methylpiperazino, N-Thiomorpholino oder N-Dioxothiomorpholino, steht, und
- R² und R⁴: für geradkettiges oder verzweigtes Alkyl mit bis zu 4 Kohlenstoffatomen, insbesondere Methyl, stehen, und
- R³: für geradkettiges oder verzweigtes Alkyl mit bis zu 4 Kohlenstoffatomen, insbesondere Methyl, oder für einen Benzylrest steht, der gegebenenfalls durch Reste aus der Reihe Wasserstoff, geradkettiges oder verzweigtes Alkyl mit bis zu 4 Kohlenstoffatomen, insbesondere Methyl, Ethyl, Propyl, Isopropyl, Butyl, Isobutyl, sec-Butyl, tert-Butyl, cyclisches Alkyl mit bis zu 6 Kohlenstoffatomen, insbesondere Cyclopropyl, Cyclobutyl, Cyclopentyl, Cyclohexyl, Alkoxy, insbesondere Methoxy, Ethoxy, Propoxy, Isopropoxy, Butoxy, Isobutoxy, sec-Butoxy, tert-Butoxy, Cycloalkoxy, insbesondere Cyclopropyloxy, Alkenyloxy, insbesondere Allyloxy, Dioxoalkylen, insbesondere Dioxomethylen, Alkylamino, insbesondere Methylamino, Ethylamino, Dialkylamino, insbesondere Dimethylamino, Diethylamino, Alkoxycarbonyl, insbesondere Methoxycarbonyl, Ethoxycarbonyl, tertButoxycarbonyl, Alkylaminocarbonyl, insbesondere Methylaminocarbonyl, Dialkylaminocarbonyl, insbesondere Dimethylaminocarbonyl, Alkylaminosulfonyl, insbesondere Methylaminosulfonyl, Dialkylaminosulfonyl, insbesondere Dimethylaminosulfonyl, Alkylthio, insbesondere Methylthio oder tert-Butylthio, Alkylsulfinyl, insbesondere Methylsulfinyl oder tert-Butylsulfinyl, Alkylsulfonyl, insbesondere Methylsulfonyl oder tert-Butylsulfonyl, Heteroarylsulfonyl, insbesondere N-Morpholinosulfonyl oder N-Pyrazolylsulfonyl, die gegebenenfalls substituiert sein können, Hydroxy, Halogen, insbesondere Brom, Chlor, Fluor oder Iod, Nitro, Amino, Carboxyl, Carbamoyl, Cyano oder der gegebenenfalls durch eine geeignete cyclische Aminogruppe, insbesondere N-Pyrrolidino, N-Piperidino, NMorpholino, N-(2,6-Dimethyl-morpholino), N-Methylpiperazino, N-Thiomorpholino oder N-Dioxothiomorpholino, substituiert ist, steht,
mit Ausnahme der Verbindungen der Formel (I), in welcher
- R¹: für 4-Hydroxy steht,
- R³: für unsubstituiertes Benzyl steht
und die übrigen Reste die oben genannten Bedeutungen haben.

Besonders bevorzugt werden Verbindungen der allgemeinen Formel (I) hergestellt,
in welcher
- R¹: für Alkoxy, insbesondere Methoxy, Ethoxy, Propoxy, Isopropoxy, Butoxy, Isobutoxy, sec-Butoxy, tert-Butoxy, Alkylamino, insbesondere Methylamino, Ethylamino, Dialkylamino, insbesondere Dimethylamino, Diethylamino, Hydroxy, Halogen, insbesondere Brom, Chlor, Fluor oder Iod, Nitro, Amino, gegebenenfalls für substituierte cyclische Aminogruppen, insbesondere N-Morpholino, N-Methylpiperazino oder N-Dioxothiomorpholino, steht, und
- R² und R⁴: für geradkettiges oder verzweigtes Alkyl mit bis zu 4 Kohlenstoffatomen, insbesondere Methyl, stehen, und
- R³: für geradkettiges oder verzweigtes Alkyl mit bis zu 4 Kohlenstoffatomen, insbesondere Methyl, oder für einen Benzylrest steht, der gegebenenfalls durch Reste aus der Reihe Wasserstoff, Alkoxy, insbesondere Methoxy, Ethoxy, Propoxy, Isopropoxy, Butoxy, Isobutoxy, sec-Butoxy, tert-Butoxy, Alkylamino, insbesondere Methylamino, Ethylamino, Dialkylamino, insbesondere Dimethylamino, Diethylamino, Hydroxy, Halogen, insbesondere Brom, Chlor, Fluor oder Iod, Nitro, Amino oder der gegebenenfalls durch eine geeignete cyclische Aminogruppe, insbesondere N-Morpholino, N-Methylpiperazino oder N-Dioxothiomorpholino, substituiert ist, steht,
mit Ausnahme der Verbindungen der Formel (I), in welcher
- R¹: für 4-Hydroxy steht,
- R³: für unsubstituiertes Benzyl steht
und die übrigen Reste die oben angegebene Bedeutung haben.

Ganz besonders bevorzugt werden Verbindungen der allgemeinen Formel (I) hergestellt,
in welcher
- R¹: für Alkoxy, insbesondere Methoxy, tert-Butoxy, Hydroxy, Halogen, insbesondere Brom, Chlor, Fluor oder Iod, Nitro, Amino, Dialkylamino, insbesondere Dimethylamino, oder gegebenenfalls für substituierte cyclische Aminogruppen, insbesondere N-Morpholino, steht, und
- R² und R⁴: für Methyl stehen, und
- R³: für einen Benzylrest steht, der gegebenenfalls durch Reste aus der Reihe Wasserstoff, Alkoxy, insbesondere Methoxy, tert-Butoxy, Hydroxy, Halogen, insbesondere Brom, Chlor, Fluor oder Iod, Nitro, Amino, Dialkylamino, insbesondere Dimethylamino, oder der gegebenenfalls durch eine geeignete cyclische Aminogruppe, insbesondere N-Morpholino, substituiert ist, steht,
mit Ausnahme der Verbindungen der Formel (I), in welcher
- R¹: für 4-Hydroxy steht,
- R³: für unsubstituiertes Benzyl steht
und die übrigen Reste die oben angegebene Bedeutung haben.

Unter den erfindungsgemäßen Herstellungsbedingungen entstehen die optisch aktiven, stereoisomeren Formen der Verbindungen der allgemeinen Formel (I). Vorzugsweise werden jedoch Verbindungen der allgemeinen Formel (I) gebildet, in denen die α-Aminosäuren eine (S)-Konfiguration (L-Form) und die 2-Hydroxycarbonsäuren eine (R)-Konfiguration (D-Form) aufweisen.

Im einzelnen seien folgende optisch aktiven Verbindungen der allgemeinen Formel (I) genannt:
Cyclo(-N-methyl-L-leucyl-D-4-nitro-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-nitro-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-nitro-phenyllactyl-N-methyl-L-leucyl-D-lactyl-N-methyl-L-leucyl-D-4-nitro-phenyllactyl-N-methyl-L-leucyl-D-lactyl-)
Cyclo(-N-methyl-L-leucyl-D-3-nitro-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-nitro-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-nitro-phenyllactyl-N-methyl-L-leucyl-D-lactyl-N-methyl-L-leucyl-D-3-nitro-phenyllactyl-N-methyl-L-leucyl-D-lactyl-)
Cyclo(-N-methyl-L-leucyl-D-2-nitro-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-nitro-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-nitro-phenyllactyl-N-methyl-L-leucyl-D-lactyl-N-methyl-L-leucyl-D-2-nitro-phenyllactyl-N-methyl-L-leucyl-D-lactyl-)
Cyclo(-N-methyl-L-leucyl-D-4-amino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-amino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-amino-phenyllactyl-N-methyl-L-leucyl-D-lactyl-N-methyl-L-leucyl-D-4-amino-phenyllactyl-N-methyl-L-leucyl-D-lactyl)
Cyclo(-N-methyl-L-leucyl-D-3-amino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-amino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-amino-phenyllactyl-N-methyl-L-leucyl-D-lactyl-N-methyl-L-leucyl-D-3-amino-phenyllactyl-N-methyl-L-leucyl-D-lactyl)
Cyclo(-N-methyl-L-leucyl-D-2-amino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-amino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-amino-phenyllactyl-N-methyl-L-leucyl-D-lactyl-N-methyl-L-leucyl-D-2-amino-phenyllactyl-N-methyl-L-leucyl-D-lactyl-)
Cyclo(-N-methyl-L-leucyl-D-4-hydroxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-hydroxy-phenyllactyl-N-methyl-L-leucyl-D-lactyl-N-methyl-L-leucyl-D-4-hydroxy-phenyllactyl-N-methyl-L-leucyl-D-lactyl-)
Cyclo(-N-methyl-L-leucyl-D-3-hydroxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-hydroxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-hydroxy-phenyllactyl-N-methyl-L-leucyl-D-lactyl-N-methyl-L-leucyl-D-3-hydroxy-phenyllactyl-N-methyl-L-leucyl-D-lactyl)
Cyclo(-N-methyl-L-leucyl-D-2-hydroxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-hydroxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-hydroxy-phenyllactyl-N-methyl-L-leucyl-D-lactyl-N-methyl-L-leucyl-D-2-hydroxy-phenyllactyl-N-methyl-L-leucyl-D-lactyl-)
Cyclo(-N-methyl-L-leucyl-D-4-methoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-methoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-methoxy-phenyllactyl-N-methyl-L-leucyl-D-lactyl-N-methyl-L-leucyl-D-4-methoxy-phenyllactyl-N-methyl-L-leucyl-D-lactyl)
Cyclo(-N-methyl-L-leucyl-D-3-methoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-methoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-methoxy-phenyllactyl-N-methyl-L-leucyl-D-lactyl-N-methyl-L-leucyl-D-3-methoxy-phenyllactyl-N-methyl-L-leucyl-D-lactyl)
Cyclo(-N-methyl-L-leucyl-D-2-methoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-methoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-methoxy-phenyllactyl-N-methyl-L-leucyl-D-lactyl-N-methyl-L-leucyl-D-2-methoxy-phenyllactyl-N-methyl-L-leucyl-D-lactyl-)
Cyclo(-N-methyl-L-leucyl-D-4-tert-butoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-tert-butoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-tert-butoxy-phenyllactyl-N-methyl-L-leucyl-D-lactyl-N-methyl-L-leucyl-D-4-tert-butoxy-phenyllactyl-N-methyl-L-leucyl-D-lactyl)
Cyclo(-N-methyl-L-leucyl-D-3-tert-butoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-tert-butoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-tert-butoxy-phenyllactyl-N-methyl-L-leucyl-D-lactyl-N-methyl-L-leucyl-D-3-tert-butoxy-phenyllactyl-N-methyl-L-leucyl-D-lactyl)
Cyclo(-N-methyl-L-leucyl-D-2-tert-butoxy-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-tert-butoxy-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-tert-butoxy-phenyllactyl-N-methyl-L-leucyl-D-lactyl-N-methyl-L-leucyl-D-2-tert-butoxy-phenyllactyl-N-methyl-L-leucyl-D-lactyl-)
Cyclo(-N-methyl-L-leucyl-D-4-N-morpholino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-N-morpholino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-N-morpholino-phenyllactyl-N-methyl-L-leucyl-D-lactyl-N-methyl-L-leucyl-D-4-N-morpholino-phenyllactyl-N-methyl-L-leucyl-D-lactyl)
Cyclo(-N-methyl-L-leucyl-D-3-N-morpholino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-N-morpholino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-N-morpholino-phenyllactyl-N-methyl-L-leucyl-D-lactyl-N-methyl-L-leucyl-D-3-N-morpholino-phenyllactyl-N-methyl-L-leucyl-D-lactyl)
Cyclo(-N-methyl-L-leucyl-D-2-N-morpholino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-N-morpholino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-N-morpholino-phenyllactyl-N-methyl-L-leucyl-D-lactyl-N-methyl-L-leucyl-D-2-N-morpholino-phenyllactyl-N-methyl-L-leucyl-D-lactyl-)
Cyclo(-N-methyl-L-leucyl-D-4-dimethylamino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-dimethylamino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-dimethylamino-phenyllactyl-N-methyl-L-leucyl-D-lactyl-N-methyl-L-leucyl-D-4-dimethylamino-phenyllactyl-N-methyl-L-leucyl-D-lactyl)
Cyclo(-N-methyl-L-leucyl-D-3-dimethylamino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-dimethylamino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-dimethylamino-phenyllactyl-N-methyl-L-leucyl-D-lactyl-N-methyl-L-leucyl-D-3-dimethylamino-phenyllactyl-N-methyl-L-leucyl-D-lactyl)
Cyclo(-N-methyl-L-leucyl-D-2-dimethylamino-phenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-dimethylamino-phenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-dimethylamino-phenyllactyl-N-methyl-L-leucyl-D-lactyl-N-methyl-L-leucyl-D-2-dimethylamino-phenyllactyl-N-methyl-L-leucyl-D-lactyl-)
Cyclo(-N-methyl-L-leucyl-D-4-iodphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-iodphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-iodphenyllactyl-N-methyl-L-leucyl-D-lactyl-N-methyl-L-leucyl-D-4-iodphenyllactyl-N-methyl-L-leucyl-D-lactyl)
Cyclo(-N-methyl-L-leucyl-D-3-iodphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-iodphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-iodphenyllactyl-N-methyl-L-leucyl-D-lactyl-N-methyl-L-leucyl-D-3-iodphenyllactyl-N-methyl-L-leucyl-D-lactyl)
Cyclo(-N-methyl-L-leucyl-D-2-iodphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-iodphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-iodphenyllactyl-N-methyl-L-leucyl-D-lactyl-N-methyl-L-leucyl-D-2-iodphenyllactyl-N-methyl-L-leucyl-D-lactyl-)
Cyclo(-N-methyl-L-leucyl-D-4-bromphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-bromphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-bromphenyllactyl-N-methyl-L-leucyl-D-lactyl-N-methyl-L-leucyl-D-4-bromphenyllactyl-N-methyl-L-leucyl-D-lactyl)
Cyclo(-N-methyl-L-leucyl-D-3-broinphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-bromphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-bromphenyllactyl-N-methyl-L-leucyl-D-lactyl-N-methyl-L-leucyl-D-3-bromphenyllactyl-N-methyl-L-leucyl-D-lactyl)
Cyclo(-N-methyl-L-leucyl-D-2-bromphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-bromphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-bromphenyllactyl-N-methyl-L-leucyl-D-lactyl-N-methyl-L-leucyl-D-2-bromphenyllactyl-N-methyl-L-leucyl-D-lactyl-)
Cyclo(-N-methyl-L-leucyl-D-4-chlorphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-4-chlorphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-4-chlorphenyllactyl-N-methyl-L-leucyl-D-lactyl-N-methyl-L-leucyl-D-4-chlorphenyllactyl-N-methyl-L-leucyl-D-lactyl)
Cyclo(-N-methyl-L-leucyl-D-3-chlorphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-3-chlorphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-3-chlorphenyllactyl-N-methyl-L-leucyl-D-lactyl-N-methyl-L-leucyl-D-3-chlor-phenyllactyl-N-methyl-L-leucyl-D-lactyl)
Cyclo(-N-methyl-L-leucyl-D-2-chlorphenyllactyl-N-methyl-L-leucyl-D-lactyl-N-methyl-L-leucyl-D-lactyl-N-methyl-L-leucyl-D-lactyl-)
Cyclo(-N-methyl-L-leucyl-D-2-bromphenyllactyl-N-methyl-L-leucyl-D-lactyl-N-methyl-L-leucyl-D-phenyllactyl-N-methyl-L-leucyl-D-lactyl-)
Cyclo(-N-methyl-L-leucyl-D-2-chlorphenyllactyl-N-methyl-L-leucyl-D-lactyl-N-methyl-L-leucyl-D-2-chlorphenyllactyl-N-methyl-L-leucyl-D-lactyl-)

Die Verbindungen der allgemeinen Formel (I) sind teilweise bekannt (vgl. z.B.: WO 94/19334; WO 95/19053; EP-OS 634 408; EP-OS 626 375; EP-OS 626 376).

Setzt man bei dem erfindungsgemäßen Verfahren a zur Herstellung der substituierten Arylmilchsäure-haltigen Cyclodepsipeptide mit 24 Ringatomen (I) als Aminosäuren der Formel (II) 4-Nitrophenyl-alanin (R¹ = 4-NO₂) ein, so läßt sich das Verfahren z.B. durch folgendes Reaktionsschema wiedergeben:

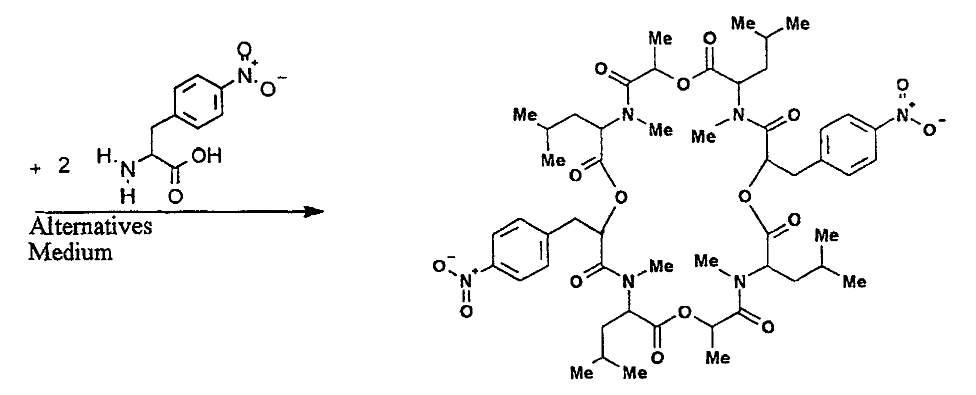

Die zur Durchführung des erfindungsgemäßen Verfahrens a als Ausgangsstoffe benötigten Aminosäuren sind durch die Formeln (II) bis (V) allgemein definiert. In diesen Formeln stehen R¹, R², R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten natürlichen oder synthetischen Aminosäuren können, falls chiral, in der (S)- oder (R)-Konfiguration (L- oder D-Form) vorliegen. Bevorzugt sind jedoch (S)-konfigurierte α-Aminosäuren.

Beispielsweise seien genannt:
Aad, Abu, jAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, hLys, hMet, hPhe, hPro, hSer, hThr, hTrp, hTyr, HyI, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Nal, Tbg, Npg, Chg, Thia (vgl. z.B. Houben-Weyl, Methoden der Organischen Chemie, Band XV/1 und 2, Stuttgart, 1974).

Bevorzugt seien jedoch substituierte Phenylalanine (Phe) genannt. In den substituierten Phenylalaninen der allgemeinen Formel (II) steht R¹ vorzugsweise für denjenigen Rest, der bereits im Zusammenhang mit der Beschreibung der erfindungsgemäßen Stoffe der Formel (I) als bevorzugt für diesen Substituenten genannt wurde.

Setzt man bei dem erfindungsgemäßen Verfahren b zur Herstellung der substituierten Arylmilchsäure-haltigen Cyclodepsipeptide mit 24 Ringatomen (I) als 2-Hydroxycarbonsäuren der Formel (VI) 4-Nitrophenyl-milchsäure (R¹ = 4-NO₂) ein, so läßt sich das Verfahren b z. B. durch folgendes Reaktionsschema wiedergeben:

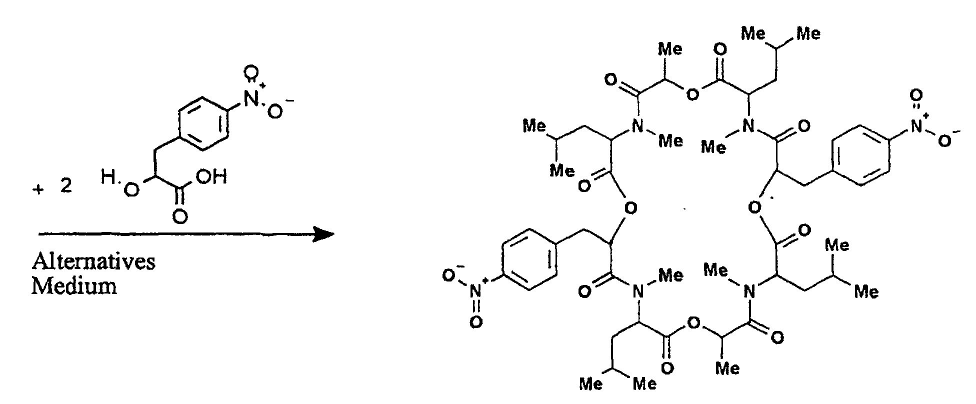

Die zur Durchführung des erfindungsgemäßen Verfahrens b als Ausgangsstoffe benötigten 2-Hydroxy-carbonsäuren sind durch die Formeln (VI) bis (IX) allgemein definiert.

In diesen Formeln stehen R¹, R², R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten 2-Hydroxy-carbonsäuren können, falls chiral, in der (S)- oder (R)-Konfiguration (L- oder D-Form) vorliegen. Bevorzugt sind jedoch die (S)-konfigurierten 2-Hydroxy-carbonsäuren.

Beispielsweise seien genannt:
Hyac, Hyba, Hydd, Hyde, Hyic, Hyiv, Hymb, Hypp, Hypr (Lac), Hytd, Hyud, Hyva, PhLac (vgl. z.B. Houben-Weyl, Methoden der Organischen Chemie, Band XV/1 und 2, Stuttgart, 1974).

Bevorzugt seien jedoch substituierte Phenylmilchsäuren (PhLac) genannt. In den substituierten Phenylmilchsäuren der allgemeinen Formel (VI) steht R¹ vorzugsweise für denjenigen Rest, der bereits im Zusammenhang mit der Beschreibung der erfindungsgemäßen Stoffe der Formel (I) als bevorzugt für diesen Substituenten genannt wurde.

Als geeigneter Pilzstamm zur Durchführung der beiden erfindungsgemäßen Verfahren a und b sei Mycelia sterilia der Art Agonomycetales genannt.

Insbesondere sei der am 30.11.1995 unter der Nummer DSM 10 345 bei der Deutschen Sammlung für Mikroorganismen (DMS) in Braunschweig gemäß Budapester Vertrag hinterlegte Pilzstamm Mycelia sterilia 541-11 genannt.

Das Verfahren kann auch mit aus Mikroorganismen isolierten Synthetasen durchgeführt werden. Die dafür benötigten Cyclodepsipeptid-Synthetasen können aus dem weiter oben genannten Pilzstamm nach literaturbekannten Verfahren isoliert werden (vgl. z. B. Isolierung von Enniatinsynthetasen: R. Pieper, H. Kleinkauf, R. Zocher, J. Antibiotics 45, 1993, S. 1273-1277; DE-A 4 406 025).

Die Fermentation der Pilzstämme der Art Agonomycetales erfolgt nach an sich bekannten Methoden in Gegenwart geeigneter Nährlösungen. Diese Nährlösungen enthalten die für das Wachstum der Pilze notwendigen Salze sowie Kohlenstoffund Stickstoffquellen.

Als geeignete organische Salze zur Durchführung des erfindungsgemäßen Verfahrens kommen alle Alkali-, Erdalkali- und Metall-Salze mit Elementen der II. bis VIII. Nebengruppe des Periodensystems in Frage.

Beispielhaft seien dafür erwähnt die Acetate, Chloride, Bromide, Iodide, Fluoride, Nitrate, Nitrite, Phosphate, Hydrogenphosphate, Dihydrogenphosphate, Phosphite, Hydrogenphosphite, Sulfate, Hydrogensulfate, Sulfite, Hydrogensulfite, Karbonate, Hydrogenkarbonate des Lithiums, Natriums, Kaliums, Caesiums, Magnesiums, Calziums, Bariums, Zinks, Cadmiums, Scandiums, Titans, Zirkoniums, Vanadiums, Niobs, Chroms, Molybdäns, Mangans, Eisen, Cobalts oder Nickels.

Vorzugsweise verwendet man die Acetate, Halogenide, Phosphate, Hydrogenphosphate, Dihydrogenphosphate, und Nitrate der Erdalkalimetalle, insbesondere Magnesium, und Metalle der II., VII. und VIII. Nebengruppe des Periodensystems, beispielsweise Zink, Mangan und Eisen.

Als Kohlenstoffquelle zur Durchführung des erfindungsgemäßen Verfahrens kommen Kohlenhydrate und kohlenhydrathaltige Produkte in Frage.

Beispielhaft seien dafür erwähnt die Monosaccharide wie Pentosen, insbesondere Ribose, die Hexosen, insbesondere Glucose und Fructose, die Oligosaccharide wie Disaccharide, insbesondere Saccharose, Maltose und Lactose, die Trisaccharide, insbesondere Raffinose, sowie die Tetra-, Penta- und Hexasaccharide.

Vorzugsweise verwendet man Monosaccharide wie beispielsweise Hexosen, insbesondere Glucose, und Oligosaccharide wie beispielsweise Disaccharide, insbesondere Saccharose.

Als Stickstoff-Quelle zur Durchführung des erfindungsgemäßen Verfahrens kommen Aminosäuren und stickstoffhaltige Salze in Frage.

Beispielhaft seien dafür die weiter oben genannten natürlichen und synthetischen Aminosäuren erwähnt oder stickstoffhaltige Salze wie Ammoniumnitrat, Ammoniumnitrit oder Nitrate und Nitrite der weiter oben genannten Metalle.

Vorzugsweise verwendet man die weiter oben genannten natürlichen Aminosäuren sowie stickstoffhaltige Salze wie Ammoniumnitrat.

Die für das fermentative Verfahren verwendeten Pilzstämme werden zunächst nach an sich bekannten Methoden in einem Medium, bestehend z. B. aus Melasse/Comsteep-Liquor, angezüchtet. Nach erfolgter Kultivation wird das Mycel isoliert. Zur Darstellung der Vorkultur wird ein Fusarium definiertes Medium (FDM), bestehend aus einer Kohlenstoff-Quelle und anorganischen Salzen, mit Mycel angeimpft und erneut fermentiert. Nach wenigen Tagen kann die FDM-Hauptkultur durch Überimpfen von je 1 ml Vorkultur hergestellt und analog fermentiert werden.

Die eigentliche Fermentation wird dann in Gegenwart von Verbindungen der Formeln (II) bis (V) oder (VI) bis (IX) durchgeführt.

Die Fermentationsdauer beträgt 1 bis 30 Tage. Die Fermentation erfolgt bei Temperaturen zwischen +5°C und +40°C, bevorzugt zwischen +15°C und +35°C, besonders bevorzugt zwischen +25°C und +30°C. Es wird steril und unter Normaldruck gearbeitet.

Zur Durchführung werden die Verbindungen der Formeln (II) bis (V) oder (VI) bis (IX) im allgemeinen in einer Konzentration von 5 mM bis 100 mM, vorzugsweise 5 mM bis 70 mM eingesetzt.

Nach vollendeter Fermentation wird das Mycel der Kultur abgetrennt, das Filtrat gegebenenfalls mehrmals mit einem organischen Lösungsmittel extrahiert, homogenisiert und anschließend filtriert. Das anfallende Kulturfiltrat wird in üblicher Weise extrahiert, getrocknet und im Vakuum eingeengt.

Die anfallenden Cyclodepsipeptid-Rohprodukte lassen sich in üblicher Weise durch Säulenchromatographie oder durch Craig-Verteilung reinigen. Welches das optimale Verfahren ist, muß von Fall zu Fall entschieden werden. (vgl. auch die Herstellungsbeispiele).

Wird das erfindungsgemäße Verfahren in Gegenwart von isolierten Cyclodepsipeptid-Synthetasen durchgeführt, wird in einem wäßrigen Puffersystem in Gegenwart von Metallsalzen, S-Adenosyl-L-methionin (SAM) und Adenosintriphosphat (ATP) gearbeitet.

Als Metallsalze seien genannt: Acetate, Chloride, Bromide, Iodide, Fluoride, Nitrate, Phosphate, Hydrogenphosphate, Phosphite, Hydrogenphosphite, Sulfate, Hydrogensulfate, Sulfite, Hydrogensulfite, Carbonate und Hydrogencarbonate des Lithiums, Natriums, Kaliums, Caesiums. Magnesiums, Calciums oder Bariums.

Vorzugsweise verwendet man Erdalkalimetall-Salze, wie beispielsweise Magnesiumchlorid, -sulfat oder -acetat.

Die erfindungsgemäßen Verfahren a und b werden in einer wässrigen Pufferlösung durchgeführt.

Beispielhaft seien dafür kommerziell erhältliche Pufferlösungen erwähnt, z.B. für den pH-Wert 1,0, insbesondere Glycin-Salzsäure, für den pH-Wert 2,0 bis 4,0, insbesondere Citrat-Salzsäure, für den pH-Wert 5,0 bis 6,0, insbesondere Citrat-Natronlauge, für den pH-Wert 7,0, insbesondere Phosphat, für den pH-Wert 8,0, insbesondere Borat-Salzsäure, und für den pH-Wert 9,0 bis 10,0, insbesondere Borsäure/Kaliumchlorid-Natronlauge.

Vorzugsweise arbeitet man im "physiologischen Bereich", d. h. bei einem pH-Wert von 6,0 bis 9,5 und verwendet dafür bevorzugt eine Phosphatpufferlösung, insbesondere Kaliumhydrogenphosphat/Dinatriumhydrogenphosphat oder Kaliumhydrogenphosphat/Dikaliumhydrogenphosphat.

Zur Durchführung werden im allgemeinen 2 mM bis 8 mM, vorzugsweise 3 mM bis 5 mM an Verbindungen der Formeln (II) bis (V) oder (VI) bis (IX), S-Adenosyl-L-methionin (SAM), 3 mM bis 9 mM vorzugsweise 4 mM bis 6 mM Adenosintriphosphat (ATP), und 2 mM bis 25 mM, vorzugsweise 5 mM bis 15 mM an Erdalkalimetall-Salz, 10 mM bis 100 mM, vorzugsweise 40 mM bis 60 mM Puffer mit 100 µg bis 1000 µg, vorzugsweise 200 µg bis 600 µg isolierter Cyclodepsipeptid-Synthetase in-vitro eingesetzt.

Die Reaktionsdauer der enzymatischen in-vitro-Synthese beträgt 2 Minuten bis 24 Stunden. Die enzymatische in-vitro-Synthese erfolgt bei einem Temperaturbereich von 0°C bis +50°C, vorzugsweise bei +10°C bis +35°C, besonders bevorzugt zwischen +20°C und +30°C.

Sie verläuft in einem pH-Bereich von 6,5 bis 9,5, vorzugsweise bei 7,0 bis 8,0, wobei der pH-Wert durch Zugabe eines Puffers während der gesamten Reaktion bei 7,3 konstant gehalten wird.

Vorzugsweise arbeitet man unter sterilen Reaktionsbedingungen und bei Normaldruck.

Die enzymatische in-vitro-Synthese kann durch Zugabe von Wasser gestoppt werden.

Im Falle der Verwendung von Verbindungen der Formeln (II) bis (V) ist es jedoch vorteilhaft die enzymatische in-vitro-Synthese in Gegenwart der entsprechenden Transaminasen bzw. Dehydrogenasen durchzuführen.

Zur Aufarbeitung wird die wässrige Phase mehrmals mit einem organischen Lösungsmittel extrahiert, getrocknet und im Vakuum eingeengt.

Die anfallenden Cyclodepsipeptid-Rohprodukte lassen sich in üblicher Weise durch Säulenchromatographie oder durch Craig-Verteilung reinigen. Welches das optimale Verfahren ist, muß auch hier von Fall zu Fall entschieden werden (vgl. auch die Herstellungsbeispiele).

### Herstellungsbeispiele

### Beispiel 1:

### Cyclo(-N-methyl-L-leucyl-D-lactyl-N-methyl-L-leucyl-D-4-nitro-phenyllactyl-N-methyl-L-leucyl-D-lactyl-N-methyl-L-leucyl-D-4-nitro-phenyllactyl-)

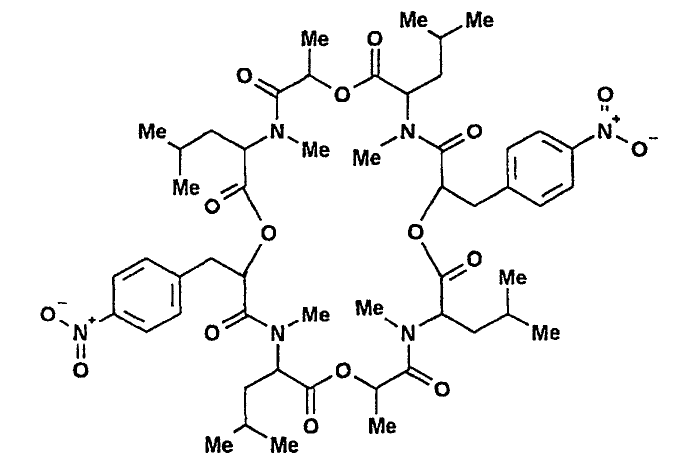

### Herstellung nach Verfahren a

### In-vivo-Incorporation von 4-Nitro-phenylalanin:

Zu einer 3 Tage alten Hauptkultur von Mycelia sterilia der Art Agonomycetales wird 4-Nitro-phenylalanin in einer Konzentration von 50 mM steril zugegeben und die Fermentation noch 3 Tage fortgesetzt. Danach wird das Mycel der Fusarienkultur abgetrennt und nach Gefriertrocknung mehrmals mit Methanol extrahiert. Anschließend werden die gesammelten organischen Phasen zur Trockne eingedampft.

### Herstellung nach Verfahren b

### In-vivo-Incorporation von 4-Nitro-phenylmilchsäure:

Zu einer 3 Tage alten Hauptkultur von Mycelia sterilia der Art Agonomycetales wird 4-Nitro-phenylmilchsäure in einer Konzentration von 50 mM steril zugegeben und die Fermentation noch 3 Tage fortgesetzt. Danach wird das Mycel der Fusarienkultur abgetrennt und nach Gefriertrocknung mehrmals mit Methanol extrahiert. Anschließend werden die gesammelten organischen Phasen zur Trockne eingedampft.

Die Aufreinigung der nach den Verfahren a und b erhaltenen Produkte erfolgt mittels präparativer HPLC (RP-18/Acetonitril-Wasser)
¹H-NMR (600 MHz, CDCl₃, δ): 7,43 (d, 8H, =CHₘₑₜₐ; 4-NO₂-Benzyl); 8,15; 8,18 (2d, 8H, =CHₒᵣₜₕₒ; 4-NO₂-Benzyl) ppm [Konformerengemisch]
APCI-MS m/z (%): 1040 (MH⁺, 13)

### In-vitro-Incorporation von 4-Nitro-phenylmilchsäure:

50 -100 µg Enzym werden in 200 µl eines Gemisches aus 4mM Dithiothreitol, 20 % Glycerin und Tris Puffer pH 7,8 für zwei Stunden bei 26°C mit den nachfolgenden Substraten inkubiert:
5 mM ATP,
10 mM MgCl₂,
2 mM L-Leucin,
0,5 mM S-Adenosyl-L-methionin,
2 mM D-Milchsäure,
50-100 µM D-4-Nitro-phenylmilchsäure.

Danach wird der gesamte Reaktionsansatz mit Essigsäureethylester extrahiert und die gebildeten Produkte werden chromatographiert (TLC, HPLC).

Analog können die in der nachstehenden Tabelle 1 aufgeführten Verbindungen der allgemeinen Formel (I) als LDLDLDLD-Stereoisomere hergestellt werden:

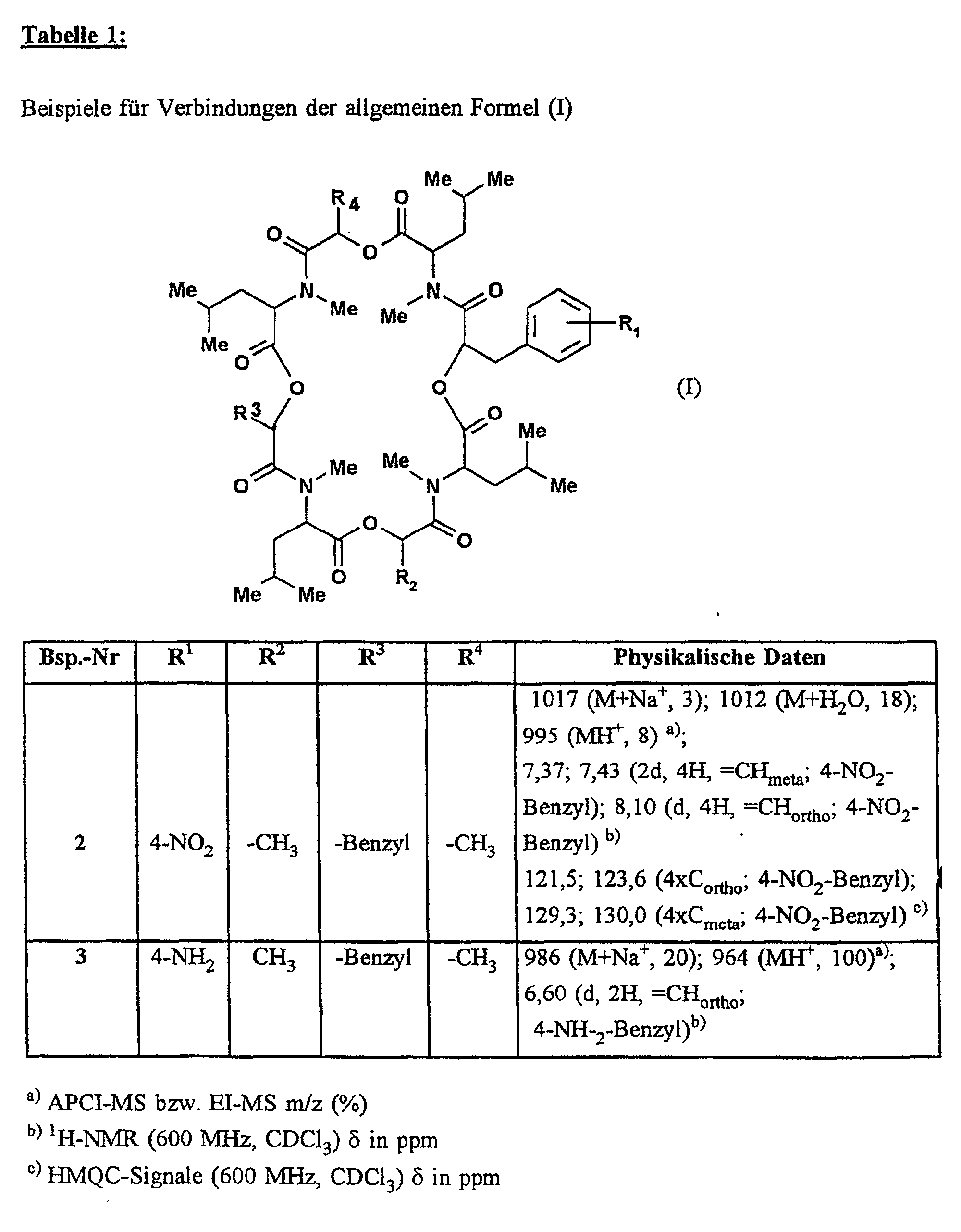

### Kultivation, Vorkulturen und Hauptkulturen

### 1. Anzucht auf Agar

Für die Anzucht der Pilzkultur auf *Agar* wird nachfolgendes Medium verwendet:
Glucose 2,0 %,
Pepton 1,0 %,
Hefeextrakt 1,0 %,
Malzextrakt 1,0 %,
KH₂PO₄ 0.05 %,
Arar 1,5 %.

Anschließend erfolgt für 20 Minuten eine Sterilisation bei 121°C.
Der pH-Wert des Mediums beträgt nach der Sterilisation ≈ 7,0.

### 2. Flüssigkultur

### a) Cornsteep / Melasse-Medium:

Der betreffende Stamm Mycelia sterilia wird in einem Medium bestehend aus . Melasse (30 g/l), Cornsteep-Liquor (10g/l) und Glucose (30 g/l) angezüchtet.

Zur Herstellung einer Vorkultur wird das Medium mit Mycel von einer Agarplatte angeimpft und nach ca. 3 Tagen auf einer Schüttelapparatur zum Animpfen für weitere Kolben (Inhalt je 100 ml) mit dem selben Medium verwendet.

### b) Alternatives Medium:

Altemativ kann für die Anzucht der Pilzkultur auch nachfolgendes Medium eingesetzt werden:
Weizenkeimextrakt 2,0 %,
Pharmamedia 1,0 %,
MgSO₄ 7 H₂O 0,2 %,
NaCl 0,2 %,
Stärke 2,6 %,
Malzsirup 6,0 %,
CaCO3 0,3 %.

Anschließend erfolgt für 20 Minuten eine Sterilisation bei 121°C.
Der pH-Wert des Mediums beträgt nach der Sterilisation 7,5.

Für die nachfolgenden Fütterungsversuche werden die betreffenden Precursoren (z.B. 4-Nitrophenylalanin, 4-Nitrophenylmilchsäure, 4-Aminophenylamin, 4-Aminophenylmilchsäure) zu 3 Tage alten Kulturen zugesetzt; die Endkonzentration beträgt 50 mM.

## Patentansprüche

1. Verfahren zur Herstellung von substituierten Arylmilchsäure-haltigen Cyclodepsipeptiden mit 24 Ringatomen der allgemeinen Formel (I) 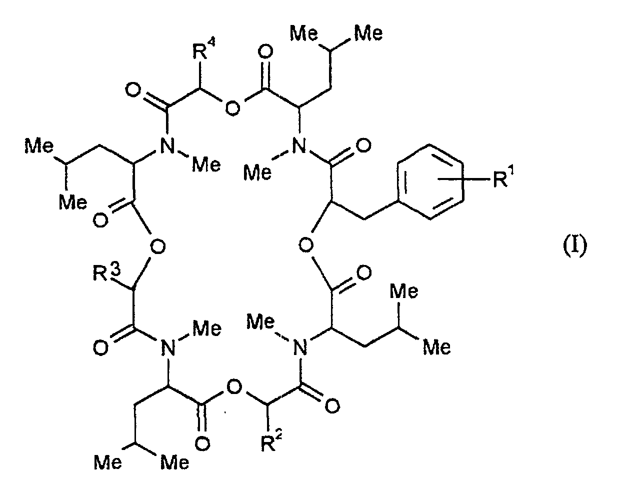 in welcher
R¹ für geradkettiges oder verzweigtes Alkyl, cyclisches Alkyl, Alkenyl, Alkoxy, Alkenyloxy, Arylalkoxy, Cycloalkoxy, Alkylamino, Dialkylamino, Alkylcarbonyl, Alkylcarbonyloxy, Alkoxycarbonyl, Alkylaminocarbonyl, Dialkylaminocarbonyl, Heteroarylcarbonyl, Alkoxysulfonyl, Alkylaminosulfonyl, Dialkylaminosulfonyl, Alkylthio, Alkylsulfinyl, Alkylsulfonyl, Heteroarylsulfonyl, die gegebenenfalls substituiert sein können, Hydroxy, Halogen, Nitro, Amino, Carboxyl, Carbamoyl, Cyano oder gegebenenfalls für substituierte cyclische Aminogruppen, steht, und
R², R³ und R⁴ unabhängig voneinander für geradkettiges oder verzweigtes Alkyl, Heteroarylmethyl oder für einen Benzylrest stehen, der gegebenenfalls durch Reste aus der Reihe Wasserstoff, geradkettiges oder verzweigtes Alkyl, cyclisches Alkyl, Alkenyl, Alkoxy, Alkenyloxy, Arylalkoxy, Cycloalkoxy, Alkylamino, Dialkylamino, Alkylcarbonyl, Alkylcarbonyloxy, Alkoxycarbonyl, Alkylaminocarbonyl, Dialkylaminocarbonyl, Heteroarylcarbonyl, Alkoxysulfonyl, Alkylaminosulfonyl, Dialkylaminosulfonyl, Alkylthio, Alkylsulfinyl, Alkylsulfonyl, Heteroarylsulfonyl, die gegebenenfalls substituiert sein können, Hydroxy, Halogen, Nitro, Amino, Carboxyl, Carbamoyl, Cyano oder der gegebenenfalls durch eine geeignete cyclische Aminogruppe substituert ist,
mit Ausnahme der Verbindungen der Formel (I), in welcher
R¹ für 4-Hydroxy steht,
R³ für unsubstituiertes Benzyl steht
und die übrigen Reste die oben genannte Bedeutung haben, **dadurch gekennzeichnet, daß** man
a) optisch aktive oder racemische Aminosäuren der allgemeinen Formeln (II), (III), (IV) und (V) 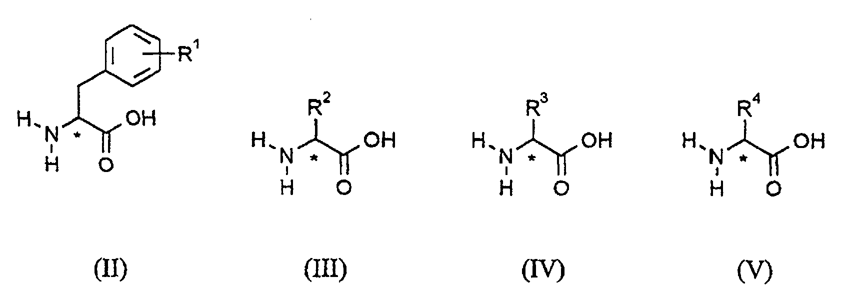 worin
R¹, R², R³ und R⁴ die oben angegebene Bedeutung haben, oder
b) optisch aktive oder racemische 2-Hydroxy-carbonsäuren der allgemeinen Formeln (VI), (VII), (VIII) und (IX) 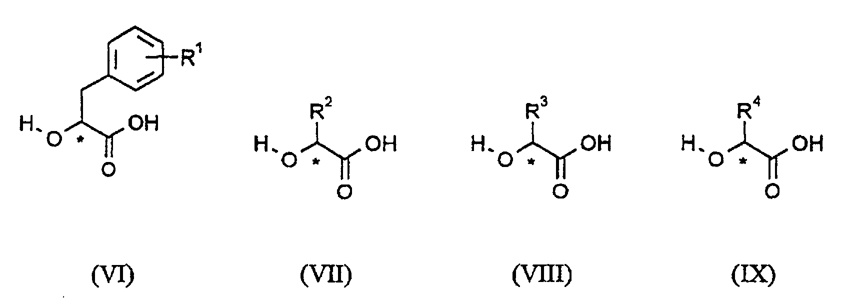 worin
R¹, R², R³ und R⁴ die oben angegebene Bedeutung haben,
in Gegenwart von Pilzstämmen der Art Agonomycetales in geeigneten Nährlösungen oder in Gegenwart von aus diesen Pilzstämmen isolierten Synthetasen in einem Puffersystem umsetzt und anschließend die gewünschten, substituierten Arylmilchsäure-haltigen Cyclodepsipeptide mit 24 Ringatomen isoliert.

## Claims

1. Process for the preparation of substituted aryllactic acid-containing cyclodepsipeptides having 24 ring atoms of the general formula (I) 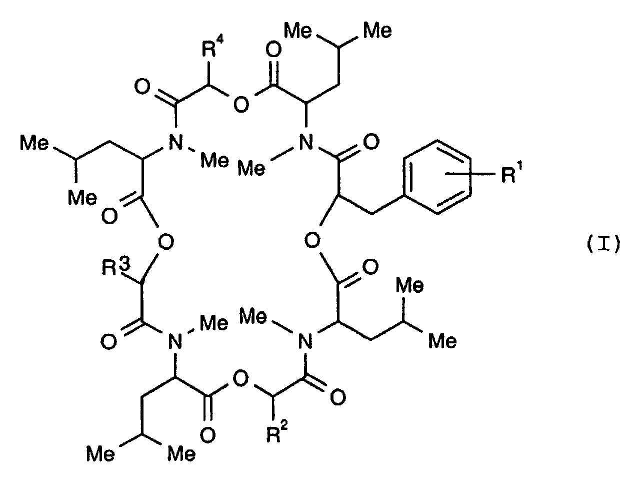 in which
R¹ represents straight-chain or branched alkyl, cyclic alkyl, alkenyl, alkoxy, alkenyloxy, arylalkoxy, cycloalkoxy, alkylamino, dialkylamino, alkylcarbonyl, alkylcarbonyloxy, alkoxycarbonyl, alkylaminocarbonyl, dialkylaminocarbonyl, heteroarylcarbonyl, alkoxysulfonyl, alkylaminosulfonyl, dialkylaminosulfonyl, alkylthio, alkylsulfinyl, alkylsulfonyl, heteroarylsulfonyl, each of which can optionally be substituted, hydroxyl, halogen, nitro, amino, carboxyl, carbamoyl, cyano, or, if appropriate, substituted cyclic amino groups, and
R², R³ and R⁴ independently of one another represent straight-chain or branched alkyl, heteroarylmethyl or a benzyl radical which is optionally substituted by radicals from the series consisting of hydrogen, straight-chain or branched alkyl, cyclic alkyl, alkenyl, alkoxy, alkenyloxy, arylalkoxy, cycloalkoxy, alkylamino, dialkylamino, alkylcarbonyl, alkylcarbonyloxy, alkoxycarbonyl, alkylaminocarbonyl, dialkylaminocarbonyl, heteroarylcarbonyl, alkoxysulfonyl, alkylaminosulfonyl, dialkylaminosulfonyl, alkylthio, alkylsulfinyl, alkylsulfonyl, heteroarylsulfonyl, each of which can optionally be substituted, hydroxyl, halogen, nitro, amino, carboxyl, carbamoyl, cyano, or which is optionally substituted by a suitable cyclic amino group,
with the exception of the compounds of the formula (I) in which
R¹ represents 4-hydroxyl,
R³ represents unsubstituted benzyl
and the other radicals have the abovementioned meaning, **characterized in that**
a) optically active or racemic amino acids of the general formulae (II), (III), (IV) and (V) 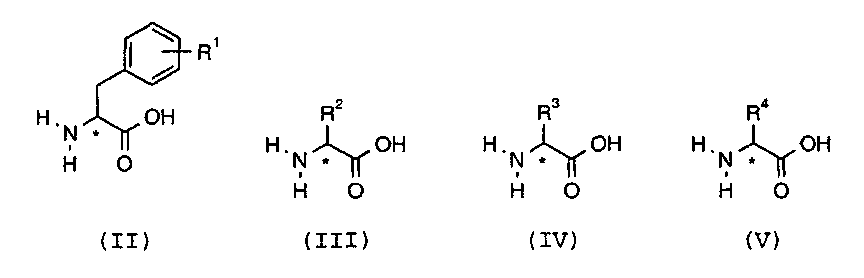 in which
R¹, R², R³ and R⁴ have the meaning given above, or
b) optically active or racemic 2-hydroxy-carboxylic acids of the general formulae (VI), (VII), (VIII) and (IX) 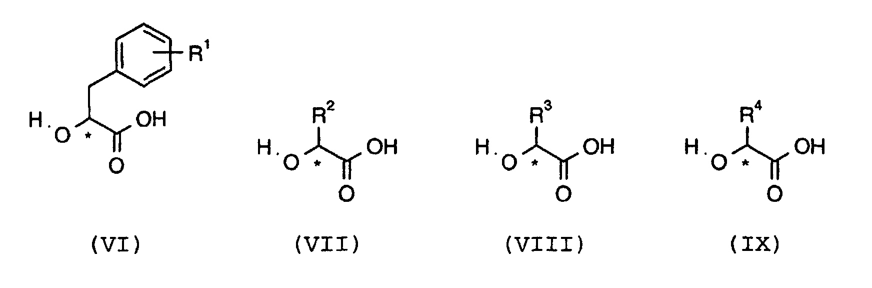 in which
R¹, R², R³ and R⁴ have the meaning given above,
are reacted in the presence of fungal strains of the species Agonomycetales in suitable nutrient solutions or in the presence of synthetases isolated from these fungal strains in a buffer system and the desired, substituted aryllactic acid-containing cyclodepsipeptides having 24 ring atoms are then isolated.

## Revendications

1. Procédé de préparation de cyclodepsipeptides substitués contenant de l'acide aryllactique, ayant 24 atomes cycliques, de la formule générale (I) : 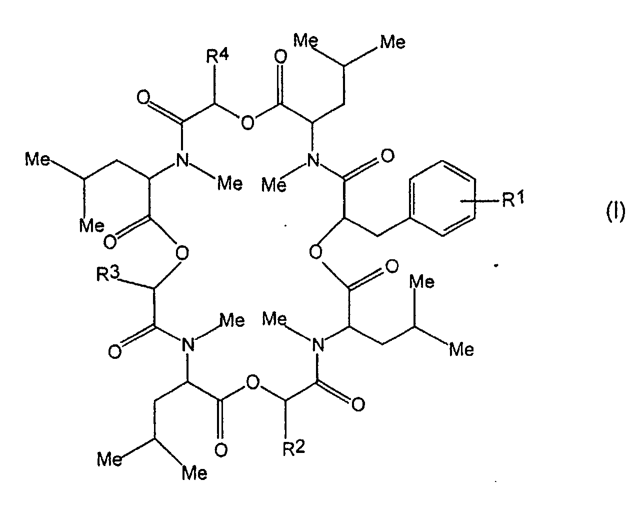 dans laquelle :
R¹ représente un radical alcoyle linéaire ou ramifié, alcoyle cyclique, alcényle, alcoxy, alcényloxy, arylalcoxy, cycloalcoxy, alcoylamino, dialcoylamino, alcoylcarbonyle, alcoylcarbonyloxy, alcoxycarbonyle, alcoylaminocarbonyle, dialcoylaminocarbonyle, hétéroarylcarbonyle, alcoxysulfonyle, alcoylaminosulfonyle, dialcoylaminosulfonyle, alcoylthio, alcoylsulfinyle, alcoylsulfonyle, hétéroarylsulfonyle, qui peuvent être, le cas échéant, substitués, hydroxy, halogène, nitro, amino, carboxyle, carbamoyle, cyano ou le cas échéant, le radical amino cyclique substitué, et
R², R³ et R⁴ représentent indépendament l'un de l'autre, un radical alcoyle linéaire ou ramifié, hétéroarylméthyle ou un reste benzyle, qui est substitué le cas échéant, par un reste de la série consistant en l'atome d'hydrogène, un radical alcoyle linéaire ou ramifié, alcoyle cyclique, alcényle, alcoxy, alcényloxy, arylalcoxy, cycloalcoxy, alcoylamino, dialcoylamino, alcoylcarbonyle, alcoylcarbonyloxy, alcoxycarbonyle, alcoylaminocarbonyle, dialcoylaminocarbonyle, hétéroarylcarbonyle, alcoxysulfonyle, alcoylaminosulfonyle, dialcoylaminosulfonyle, alcoylthio, alcoylsulfinyle, alcoylsulfonyle, hétéroarylsulfonyle, qui peuvent être, le cas échéant, substitués, hydroxy, halogène, nitro, amino, carboxyle, carbamoyle, cyano ou le cas échéant, le radical amino cyclique substitué,
à l'exception des composés de formule (I), dans lesquels :
R¹ représente le radical 4-hydroxy ;
R³ représente le radical benzyle non substitué, et
les autres restes ont la signification indiquée ci-dessus, **caractérisé en ce que** l'on fait réagir
a) les acides aminés optiquement actifs ou racémiques des formules générales (II), (III), (IV) et (V) : 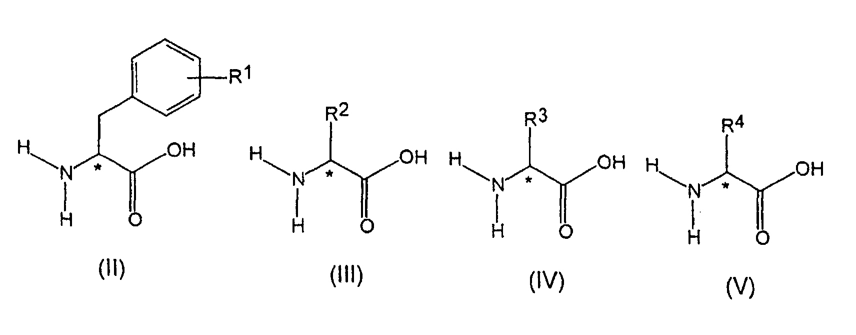 où
R¹, R², R³ et R⁴ ont la signification indiquée ci-dessus, ou
b) les acides 2-hydroxycarboxyliques optiquement actifs ou racémiques des formules générales (VI), (VII), (VIII) et (IX) : 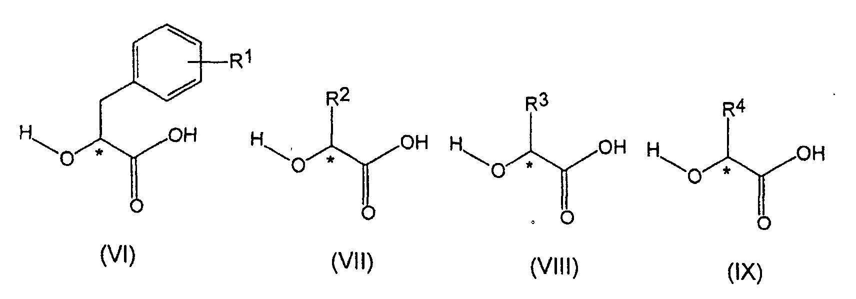 où
R¹, R², R³ et R⁴ ont la signification indiquée ci-dessus,
en présence de souches de champignons du type des Agonomycétales dans des solutions nutritives appropriées ou en présence de synthétases isolées de ces souches de champignons, dans un système tampon, et ensuite on isole le cyclodepsipeptide substitué contenant de l'acide aryllactique, ayant 24 atomes cycliques, désiré.
